# EUROPEAN PATENT APPLICATION

(11) **EP 4 197 457 A1**
(43) Date of publication of application: **21.06.2023**
(21) Application number: 21860184.7
(22) Date of filing: 13.08.2021
(51) Int. Cl.: A61B 17/12

(54) **HEMANGIOMA OCCLUSION APPARATUS, HEMANGIOMA OCCLUSION TREATMENT APPARATUS, AND HEMANGIOMA OCCLUSION SYSTEM**

(30) Priority: 31.08.2020 CN 202010899258
(71) Applicant: MicroPort NeuroTech (Shanghai) Co., Ltd., Shanghai 201318 (CN)
(72) Inventor: GUO, Shuang, Shanghai 201318 (CN); CHANG, Mengqi, Shanghai 201318 (CN); CHEN, Bing, Shanghai 201318 (CN); GUO, Yuanyi, Shanghai 201318 (CN); DENG, Shuhao, Shanghai 201318 (CN); LU, Huina, Shanghai 201318 (CN)
(74) Representative: Marks & Clerk LLP
(86) International application number: PCT/CN2021/112628
(87) International publication number: WO 2022/042346

(57) **Abstract**

An aneurysm occlusion device (10), a therapeutic apparatus for aneurysm occlusion and an aneurysm occlusion system. The aneurysm occlusion device (10) includes an expandable structure (11) and a guide structure (12). The expandable structure (11) is a three-dimensional mesh. The expandable structure (11) has an expanded configuration, in which the expandable structure forms a spiral shape by spiraling from a distal end (111) to a proximal end (112) thereof, and a collapsed configuration for delivery into an aneurysm (30) from a blood vessel. The guide structure (12) is provided at the distal end (111) of the expandable structure (11). The proximal end (121) of the guide structure (12) is connected to the distal end (111) of the expandable structure (11). The guide structure (12) has an expanded configuration, in which the guide structure forms a spiral shape by spiraling from a distal end (122) to the proximal end (121) thereof, and a collapsed configuration for delivery into the aneurysm (30) from the blood vessel. The aneurysm occlusion device (10) provides the advantages of, among others, achieving stable and compliant packing while avoiding rupture of the aneurysm, preventing vascular embolism, improving coverage of an opening at the aneurysm neck, promoting thrombosis in the aneurysm and accelerating embolization of the aneurysm.

## Description

### TECHNICAL FIELD

The present invention relates to the technical field of medical instruments and, in particular, to an aneurysm occlusion device, a therapeutic apparatus for aneurysm occlusion and an aneurysm occlusion system.

### BACKGROUND

Intracranial aneurysms are pathological bulges in intracranial artery walls, with an incidence rate of 5% to 10%. A magnetic resonance angiography (MRA) study shows that the morbidity of unruptured aneurysms for Chinese adults aged 35-75 years is approximately 7.0%. Although subarachnoid hemorrhages caused by intracranial aneurysm ruptures account for about 5% of strokes, primary ruptures are associated with a mortality rate of 20-30%, and re-ruptures are associated with a mortality rate of as high as 60%. Aneurysm treatment relies on complete isolation of an aneurysm from blood circulation by a therapeutic method. Currently available therapeutic methods primarily include craniotomy and clipping and endovascular intervention. At present, endovascular intervention has been predominant because it allows direct access to a lesion without affecting the brain tissue and because of its minimally invasive nature. Currently available endovascular interventional therapies are principally as follows:
(1) At present, aneurysm embolization with coils is mostly used in aneurysm treatment. It can achieve aneurysm occlusion and treatment by promoting thrombosis through altering local hemodynamic factors. However, as aneurysms are diverse in shape and size, incomplete coil packing may lead to recanalization of an aneurysm, while excessive packing may cause intraoperative rupture of an aneurysm. This places stringent requirements on physicians' dexterity and experience. Moreover, coil packing may require multiple procedures and therefore lacks efficiency. In some cases, additional use of stents, balloons, and catheters may be required, leading to increased operational complexity. Further, for wide-neck aneurysms, coils tend to herniate into the parent arteries, affecting blood flow, or even causing vascular stenosis in severe cases.
(2) As a significant breakthrough in the field of endovascular treatment of intracranial aneurysms, flow diverters provide a novel approach for the treatment of complex aneurysms. This approach involves placing a dense mesh stent in a parent artery, which enables endoluminal reconstruction of the diseased blood vessel and subsequent luminal surface re-endothelialization by neointimal growth over the aneurysm neck. The use of flow diverters has significantly improved long-term therapeutic benefit for large and giant aneurysms and considerably reduced the use of coils. In addition, according to computer-aided hemodynamic simulation analysis, a metal coverage percentage of 30-50% can significantly reduce intra-aneurysmal blood flow and achieve a high cure rate. However, the use of flow diverters can lead to long-term reliance of patients on dual antiplatelet therapy and a risk of hemorrhagic complications after surgery. Further, some large aneurysms are associated with a risk of rupture a certain period of time after the treatment.
(3) Currently, there are also novel devices capable of embolization in one pass, which are generally made of shape memory materials and shaped into balls, cylinders or discs. Such a device is designed to be delivered through a catheter to a target site, where it is pushed out of a sheath and then self-expand to recover its spherical shape, thereby blocking the aneurysm. For example, a first embolization device is a spherical or cylindrical dense mesh device with anchors at its opposing ends. After full expansion of the device within an aneurysm, a proximal dense mesh thereof covers the neck of the aneurysm to therapeutically treat the aneurysm. A second embolization device is a three-dimensional mesh-like structure composed of a radiopaque wire and surrounding self-expanding shape memory alloy wires. Similar to a coil, this device can be released and retrieved by a catheter and packed within an aneurysm in a spherical form capable of disturbing blood flow. A third embolization device is a braided double-layer nickel-titanium alloy device. A fourth embolization device is a braided double-layer shape memory alloy device, which is in the form of a coiled disc when not stressed. However, when released into an aneurysm, it will be stressed by walls of the aneurysm and assume a tulip-like shape, which allows it to be stabilized in a lower part of the aneurysm while covering the aneurysm neck to provide an effect of hemodynamic remodeling. However, in the design of the first embolization device, the proximal anchor makes the device symmetric and necessary to cover the aneurysm neck in a certain orientation. Therefore, it is used mainly for the treatment of bifurcated wide-neck aneurysms and is particularly suitable for those with a regular morphology. In the design of the first embolization device, the distal anchor may impact an aneurysm wall and is likely to cause a rupture or bleeding of the aneurysm. In some cases, the proximal anchor of the first embolization device may be pushed by the aneurysm wall to herniate into the parent artery, affecting the endothelialization of the aneurysm neck. In addition, the first embolization device is generally in the form of a single ball or cylinder, which is insufficiently strong despite of a large contact area. This is unfavorable to long-term stability, and tends to lead to displacement, of the device within the aneurysm. The three-dimensional mesh-like structure of the second embolization device is spherical and composed of multiple meshed sheets. Due to significant friction between different parts of the three-dimensional mesh-like structure and between the structure itself and aneurysm walls, it is difficult for the device to return to the intended shape and, and even if this is achieved, the device cannot maintain the shape stably for a sufficiently long period of time. Therefore, it cannot provide desirable packing quality. Moreover, since this device must be used in combination with a coil, its operation is complicated. The third embolization device operates in a similar manner as the first embolization device and therefore suffers from similar problems. The fourth embolization device also has a proximal anchor, which likewise tends to be pushed by an aneurysm wall to herniate into the parent artery and makes the device only suitable for use in apex aneurysms. Moreover, as this device requires repeated relocation before it can satisfactorily stay stably within the aneurysm, it lacks efficiency.

### SUMMARY OF THE INVENTION

In order to overcome the above problems, it is an object of the present invention to provide an aneurysm occlusion device, a therapeutic apparatus for aneurysm occlusion and an aneurysm occlusion system. The aneurysm occlusion device is capable of more easily recovering, and more robustly maintaining, a preformed shape in an aneurysm and causing less damage to a wall of the aneurysm, associated with a reduced risk of rupture of the aneurysm, and able to be compressed to a smaller size enabling delivery within a catheter having a small inner diameter to a wider variety of lesions or through narrower blood vessels.

In order to achieve the above object, the present invention provides an aneurysm occlusion device provided in the present invention, which includes an expandable structure and a guide structure. The expandable structure is a three-dimensional mesh and has an expanded configuration, in which the expandable structure forms a spiral shape by spiraling from a distal end to a proximal end thereof, and a collapsed configuration for delivery into an aneurysm from a blood vessel.

The guide structure is provided at the distal end of the expandable structure and has a proximal end connected to the distal end of the expandable structure. The guide structure has an expanded configuration, in which the guide structure forms a spiral shape by spiraling from a distal end to the proximal end thereof, and a collapsed configuration for delivery into the aneurysm from the blood vessel.

Optionally, the guide structure may be an elongated member spiraling from a distal end to a proximal end thereof into a three-dimensional spiral shape.

Optionally, the guide structure may spiral in a same direction as the expandable structure.

Optionally, an axis of spiral of the guide structure may coincide with an axis of spiral of the expandable structure.

Optionally, a distal radiopaque ring may be fixedly connected to the distal end of the guide structure, and/or a proximal radiopaque ring may be fixedly connected to the proximal end of the expandable structure.

Optionally, a cross-sectional area of the expandable structure may increase and then decrease from the proximal end to the distal end thereof.

Optionally, the expandable structure may comprise a proximal section, a middle section and a distal section which are connected axially in sequence,
wherein the proximal section has a cross-sectional area gradually increasing from a proximal end to a distal end thereof, and/or the distal section has a cross-sectional area gradually increasing from a distal end to a proximal end thereof.

Optionally, a cross-sectional area of the expandable structure may repeatedly increase and then decrease from the proximal end to the distal end thereof.

Optionally, a maximum outer diameter of the expandable structure in the expanded configuration thereof may be not smaller than 1/4 of an outer diameter of a largest spiral turn in the expandable structure.

Optionally, the maximum outer diameter of the expandable structure in the expanded configuration thereof may be 1/3-1/2 of the outer diameter of the largest spiral turn in the expandable structure.

Optionally, the maximum outer diameter of the expandable structure may range from 2.0 mm to 8.0 mm, and the outer diameter of the largest spiral turn in the expandable structure may range from 3.0 mm to 25 mm.

Optionally, the expandable structure may be formed by braiding 48-144 filaments which are made of a shape memory material and have a diameter of 0.0005-0.002 inches.

Optionally, the expandable structure may be formed by braiding radiopaque filaments, or by braiding both radiopaque and non-radiopaque filaments.

Optionally, the spiral shape of the expandable structure in the expanded configuration thereof may have one spiral turn or a plurality of spiral turns,
wherein in case of the plurality of spiral turns, a first one of the spiral turns in the expandable structure proximal to the distal end thereof has an outer diameter smaller than an outer diameter of each spiral turn therein other than the first spiral turn.

Optionally, in case of the spiral shape of the expandable structure in the expanded configuration thereof having the plurality of spiral turns, the outer diameter of the first spiral turn may be not smaller than 2/3 of the outer diameter of each spiral turn other than the first spiral turn.

Optionally, in case of the spiral shape of the expandable structure in the expanded configuration thereof having the plurality of spiral turns, the outer diameter(s) of the other spiral turn(s) than the first spiral turn in the expandable structure may be equal.

Optionally, the spiral shape of the guide structure in the expanded configuration thereof may have one spiral turn or a plurality of spiral turns,
wherein in case of the guide structure having the plurality of spiral turns, a first one of the spiral turns in the guide structure proximal to the distal end thereof may have an outer diameter smaller than an outer diameter of each spiral turn therein other than the first spiral turn.

Optionally, in case of the spiral shape of the guide structure in the expanded configuration thereof having the plurality of spiral turns, the outer diameter of each spiral turn other than the first spiral turn in the guide structure may not exceed the outer diameter of the first spiral turn in the expandable structure proximal to the distal end thereof.

Optionally, in case of the spiral shape of the guide structure in the expanded configuration having the plurality of spiral turns, the outer diameter of the first spiral turn in the guide structure proximal to the distal end thereof may be not smaller than 2/3 of the outer diameter of each spiral turn therein other than the first spiral turn.

Optionally, in case of the spiral shape of the guide structure in the expanded configuration having the plurality of spiral turns, the outer diameter(s) of the other spiral turn(s) than the first spiral turn in the guide structure may be equal.

Optionally, in case of the spiral shape of the guide structure in the expanded configuration having the plurality of spiral turns, the outer diameter of each spiral turn other than the first spiral turn in the guide structure may be not smaller than 2/3 of the outer diameter of the first spiral turn in the expandable structure proximal to the distal end thereof.

Optionally, the expandable structure and the guide structure may be integrally braided.

In order to achieve the above object, the present invention provides a therapeutic apparatus for aneurysm occlusion, which is used to deliver the aneurysm occlusion device as defined above and includes a push pod coupled to the proximal end of the expandable structure.

Optionally, the push pod may extend in a direction tangential to a spiral contour of the spiral shape of the expandable structure in the expanded configuration thereof.

The above object is also attained by an aneurysm occlusion system provided in the present invention, which includes the aneurysm occlusion device as defined above and a catheter. The expandable structure is compressed in the catheter and, after being released from the catheter, recovers the expanded configuration in which the expandable structure forms the spiral shape.

Optionally, the catheter may have an inner diameter of 0.017 inches, 0.021 inches or 0.027 inches.

In order to achieve the above object, the present invention provides a method of treating an aneurysm. The aneurysm communicates, at a neck thereof, with a blood vessel. The method includes:
1) placing the aneurysm occlusion device as defined above into the aneurysm;
2) releasing the guide structure into the aneurysm so that the guide structure spirals within the aneurysm into the predetermined shape; and
3) releasing the expandable structure into the aneurysm so that, under the guidance of the guide structure, the expandable structure continues spiraling within the aneurysm in such a manner that an outer surface of the expandable structure spans over the neck of the aneurysm.

Optionally, the method may further include:
positioning the proximal end of the expandable structure between a wall of the aneurysm and the outer surface in such a manner that the proximal end of the expandable structure is oriented parallel to the aneurysm wall without herniating into the blood vessel.

In the above-discussed aneurysm occlusion device, the expandable structure is a three-dimensional mesh and has an expanded configuration, in which it assumes a spiral shape spiraling from the distal end to the proximal end thereof, and a collapsed configuration for its delivery into an aneurysm from a blood vessel. With this design, the expandable structure will encounter less friction during its release in the aneurysm's cavity, which enables the expandable structure to more robustly and easily recover the predetermined shape in the aneurysm and more easily achieve the closure of the aneurysm neck. Moreover, by virtue of the spiral shape, the distal end of the device is wrapped up or not oriented toward a wall of the aneurysm, and the proximal end of the device is compressed between an outer surface of the three-dimensional mesh structure and the wall of the aneurysm so as to be parallel to the aneurysm wall (including tangential scenarios). As a result, both the proximal and distal ends of the device will have no impact on the aneurysm wall, avoiding the risk of rupture of the aneurysm. In addition, the outer surface of the largest spiral turn in the expandable structure in the expanded configuration can better cover and close the aneurysm neck, more easily induce thrombosis in the aneurysm, and circumvent the problem that a proximal anchor of the expandable structure is positioned around a center of the aneurysm neck opening or herniate into the parent blood vessel. Thus, endothelialization of the aneurysm neck can be accelerated, and the risk of vascular stenosis can be avoided.

In the above-discussed aneurysm occlusion device, the spiral-shaped guide structure provided at the distal end of the expandable structure can guide the expandable structure to enable it to more robustly and easily recover the predetermined shape in the aneurysm and more easily achieve the occlusion of the aneurysm neck. Moreover, since the guide structure is relatively slim and flexible, it can facilitate pushing and advancement of the entire device by reducing resistance. It can also relieve tension from the expandable structure during its release, thereby reducing its impact on the aneurysm wall and additionally reducing the risk of rupture of the aneurysm. Additionally, during the delivery from the blood vessel into the aneurysm, both the expandable structure and the guide structure can be compressed into linear shapes and accommodated in a catheter. This allows the device to have a reduced size for delivery and facilitates its delivery through a catheter with a small inner diameter. In this way, it can be delivered to reach a wider variety of lesions or through narrower blood vessels, making it usable in an expanded spectrum of therapeutic applications. In particular, the three-dimensional spiral-shaped structure can be deployed without having to be oriented in a specific direction and thus can be suitably used to treat both regular and irregular aneurysms. Moreover, it can conform to different aneurysm shapes and can more easily achieve aneurysm occlusion. In addition, the aneurysm occlusion device of the present invention can achieve embolization in one pass, dispensing with the use of multiple embolization devices. Therefore, it enables higher embolization efficiency, simpler operation, less reliance on a physician's surgical experience and reduced surgical complexity and time. Furthermore, since it is intended to close the aneurysm neck opening with the outer surface of the largest spiral turn in the expandable structure, the outer surface of the largest spiral turn is designed to match an inner diameter of the aneurysm. This enables a large contact area, high support strength and stable occlusion by the aneurysm occlusion device without easy displacement. In particular, the three-dimensional mesh design allows the expandable structure to have an even larger contact area with the aneurysm wall, which enables the aneurysm occlusion device to more firmly stay within the aneurysm, additionally reduces the chance of displacement and further lowers the risk of the proximal end of the expandable structure herniating into the parent blood vessel.

In the above-discussed aneurysm occlusion device, the guide structure preferably assumes a three-dimensional spiral shape, and more preferably, the guide structure spirals in the same direction as the expandable structure. This design enables the expandable structure to more robustly and easily recover the predetermined shape in the aneurysm and more easily achieve the occlusion of the aneurysm neck. Moreover, the expandable structure is preferred to have a cross-sectional area, which increases and then decreases, or repeatedly increases and decreases, from the proximal end to the distal end thereof. This design can facilitate configuration of the expandable structure into a fusiform shape (swollen at the middle and tapering to each end), which makes it easy to compress the device into a smaller size. Moreover, it enables the device to be more flexible and pushed and advanced while experiencing less resistance and exerting a reduced impact on the aneurysm wall. Preferably, the distal end of the expandable structure is fixedly connected with a distal radiopaque ring, in particular when the guide structure is not provided. This can increase smoothness of the distal end of the expandable structure and additionally reduce damage caused to the aneurysm wall.

In the above-discussed aneurysm occlusion device, the expandable structure is a mesh structure braided from 48-144 filaments having a diameter of 0.0005-0.002 inches. In this way, denser mesh openings can be formed, which can facilitate embolization of the aneurysm, enable the aneurysm wall to be more uniformly stressed and further reduce the risk of rupture of the aneurysm.

In the above-discussed therapeutic apparatus for aneurysm occlusion, the push pod is coupled to the proximal end of the expandable structure and preferred to extend in a direction tangential to a spiral contour of the spiral shape of the expandable structure in the expanded configuration. With this design, the push pod can be manipulated to achieve covering of the aneurysm neck opening by the outer surface of the largest spiral turn in the expandable mesh structure with increased surgical accuracy and improved coverage quality.

### BRIEF DESCRIPTION OF THE DRAWINGS

Fig. 1 is a schematic diagram illustrating the structure of an aneurysm occlusion device according to a preferred embodiment of the present invention, in which a guide structure assumes a spiral shape having 1.5 spiral turns when in an expanded configuration and an expandable structure assumes a spiral shape having 1 spiral turn when in an expanded configuration and spirals in an opposite direction to the guide structure.
Fig. 2 is a schematic diagram illustrating the structure of an aneurysm occlusion device according to a preferred embodiment of the present invention, in which a guide structure assumes a spiral shape having 2 spiral turns when in an expanded configuration and an expandable structure assumes a spiral shape having 1 spiral turn when in an expanded configuration and spirals in the same direction as the guide structure.
Fig. 3 is a schematic diagram illustrating the structure of an aneurysm occlusion device according to a preferred embodiment of the present invention, in which a guide structure assumes a spiral shape having 1 spiral turn when in an expanded configuration and an expandable structure assumes a spiral shape also having 1 spiral turn when in an expanded configuration and spirals in an opposite direction to the guide structure.
Fig. 4 shows an aneurysm occlusion device according to a preferred embodiment of the present invention, in which a guide structure has been first released into an aneurysm.
Fig. 5 shows an aneurysm occlusion device according to a preferred embodiment of the present invention, which has been completely released into an aneurysm.
Fig. 6 is a partially enlarged view of an aneurysm occlusion device according to a preferred embodiment of the present invention, which is covering an aneurysm neck opening.

Throughout the figures, the same reference numbers are used to denote the same or like elements.

### DETAILED DESCRIPTION

Objects, advantages and features of the present invention will become more apparent from the following detailed description when taken in conjunction with the accompanying drawings. Note that the figures are provided in a very simplified form not necessarily drawn to exact scale for the only purpose of facilitating easy and clear description of the disclosed embodiments.

As used herein, the singular forms "a", "an" and "the" include plural referents, unless the context clearly dictates otherwise. As used herein, the term "or" is generally employed in the sense including "and/or", unless the context clearly dictates otherwise. The term "plurality" is generally employed in the sense including "two or more than two", unless the context clearly dictates otherwise. The term "several" is generally employed in the sense including "an indefinite number of', unless the context clearly dictates otherwise. The term "proximal end" generally refers to an end closer to an operator operating a medical device, and the term "distal end" generally refers to an end of the device that enters a human body first, unless the context clearly dictates otherwise.

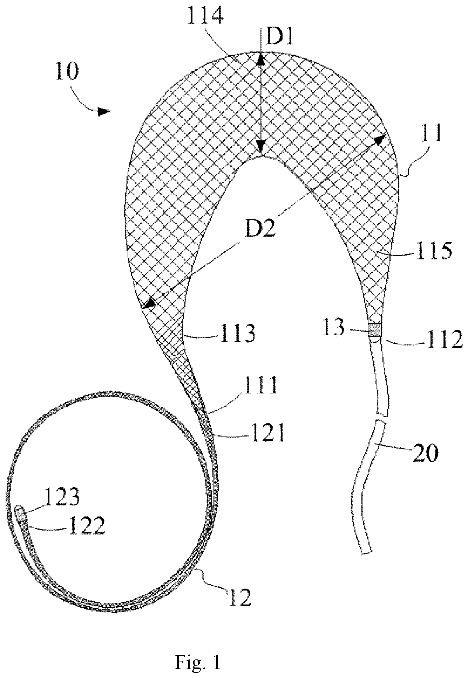

Reference is now made to Fig. 1, which shows an aneurysm occlusion device 10 according to an embodiment of the present invention. The aneurysm occlusion device 10 is used for treatment of aneurysm occlusion. Examples of the aneurysm include, but are not limited to, intracranial aneurysms. Specifically, the aneurysm occlusion device 10 includes an expandable structure 11 and a guide structure 12. The expandable structure 11 is a three-dimensional spiral-shaped mesh. In some embodiments, the three-dimensional spiral mesh does not spiral within a single horizontal plane. This design enables firmer support and strong anchoring of the expandable structure 11 within the aneurysm without easy displacement. The guide structure 12 is disposed outside the expandable structure 11 at a distal end thereof. A proximal end 121 of the guide structure 12 is joined to the distal end 111 of the expandable structure 11. The guide structure 12 assumes a spiral shape, such as a three-dimensional or planar spiral shape.

In this embodiment, the guide structure 12 is preferably composed of an elongate member, which spirals from a distal end to a proximal end thereof into a three-dimensional spiral shape. In other embodiments, the guide structure 12 may also be composed of an elongate member, which spirals from a distal end to a proximal end thereof into a planar spiral shape. The guide structure 12 may spiral in the same direction as the expandable structure 11, or not. Preferably, the guide structure 12 may spiral in the same direction as the expandable structure 11 in order to enable better guidance and avoid bending of the guide structure 12 at the distal end 122 during delivery, which may affect shape recovery of the expandable structure 11 within the aneurysm. An axis of spiral of the guide structure 12 may coincide with an axis of spiral of expandable structure 11, or not. Preferably, the axis of spiral of the guide structure 12 coincides with the axis of spiral of expandable structure 11.

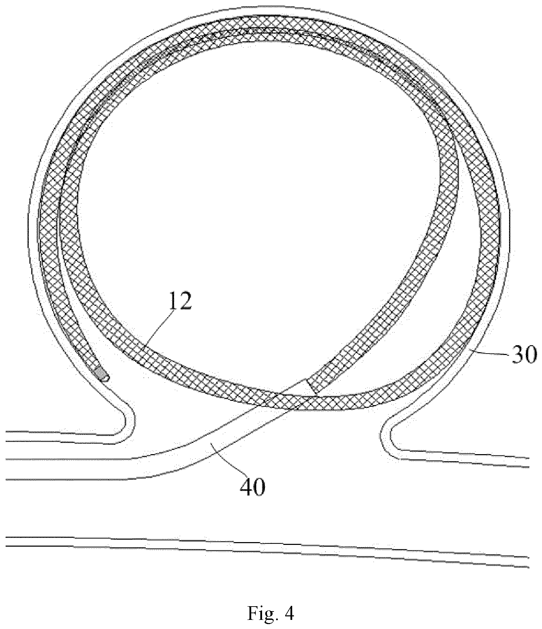

In practical use, the expandable structure 11 has a collapsed configuration and an expanded configuration. When in the expanded configuration, the expandable structure 11 assumes the spiral shape spiraling from the distal end to a proximal end thereof. The collapsed configuration of the expandable structure 11 can facilitate its delivery from a blood vessel into the aneurysm. More specifically, the expandable structure 11 assumes the collapsed configuration when loaded in a catheter 40 (which is an external mechanism, see Fig. 4). In this configuration, the expandable structure 11 may be compressed into a linear shape having a minimized radial size, which facilitates its delivery in the catheter 40 that has a small inner diameter. When released from the catheter 40, the expandable structure 11 can expand by its own resilience into the expanded configuration, in which it recovers the three-dimensional spiral shape. Likewise, the guide structure 12 also has a collapsed configuration and an expanded configuration. When in the expanded configuration, the guide structure 12 assumes the spiral shape spiraling from the distal end to the proximal end thereof, such as a three-dimensional or planar spiral shape. Similarly, the collapsed configuration of the guide structure 12 can facilitate its delivery from the blood vessel into the aneurysm. More specifically, the guide structure 12 assumes the collapsed configuration when loaded in the catheter 40. In this configuration, the guide structure 12 may be compressed into a linear shape, which facilitates its delivery in the catheter 40. When released from the catheter 40, the guide structure 12 can expand by its own resilience into the expanded configuration, in which it recovers the spiral shape.

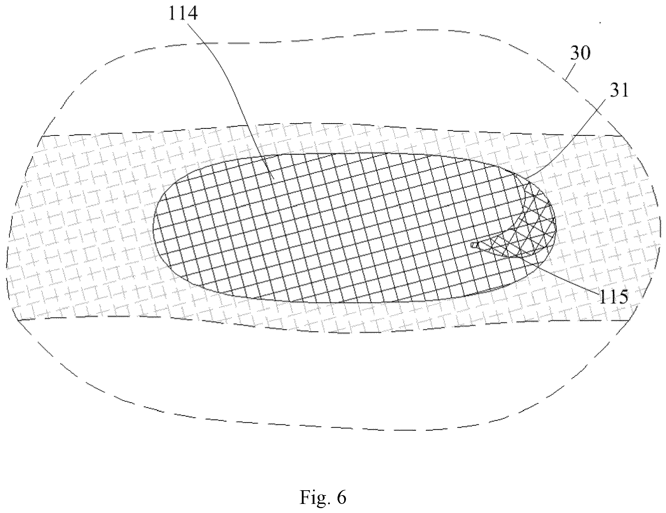

Referring back to Fig. 1, in an embodiment of the present invention, there is also provided a therapeutic apparatus for aneurysm occlusion, including a push pod 20 for delivering the aneurysm occlusion device 10. The proximal end 112 of the expandable structure 11 is configured to be detachably coupled to the push pod 20. The push pod 20 functions primarily to push and advance the aneurysm occlusion device 10 out of the catheter 40 and thereby release it into the aneurysm 30. Optionally, the proximal end 112 of the expandable structure 11 may be provided with a separate connecting means (not shown) for detachably coupling the push pod 20. Additionally, in the expanded configuration of the expandable structure 11, the push pod 20 is preferred to extend in a direction tangential to a spiral contour of the spiral shape of the expandable structure 11. In this way, the expandable structure 11 can be advanced and released so that an outer surface of its largest spiral turn covers a neck opening 31 of the aneurysm (see Fig. 6). That is, the outer surface of the three-dimensional mesh spans across the neck of the aneurysm. This enables improved coverage of the aneurysm neck opening 31 and avoids the proximal end 112 of the expandable structure 11 from being located around a center of the aneurysm neck opening 31 and thus from possibly herniating into the parent blood vessel. Thus, healing of the aneurysm neck will not be affected, and vascular embolism can be avoided. Without limiting the prevent invention, the detachment of the push pod 20 from the proximal end 112 of the expandable structure 11 may be accomplished thermally, electrically, mechanically, hydrolytically or otherwise as is conventional.

The guide structure 12 is preferred to have an elongate structure (i.e., to be an elongate member) in the expanded configuration, which has an outer diameter much smaller than an outer diameter of the three-dimensional mesh of the expandable structure 11. In this way, compared with the expandable structure 11, the guide structure 12 is more elongate and more flexible. The guide structure 12 functions mainly to: guide the expandable structure 11 by its own spiral shape to enable the expandable structure 11 to more easily recover the three-dimensional spiral shape in the aneurysm; relieve tension from the expandable structure 11 during release, thereby reducing its impact on a wall of the aneurysm; and reduce resistance to pushing and enable easier pushing. In particular, since the guide structure 12 is relatively slim and compliant, it will cause less or no damage to the aneurysm wall.

Use of the inventive aneurysm occlusion device 10 will be described below with reference to Figs. 4 to 6. However, the following description is not intended to be construed as limiting the present invention in any way. Rather, it is only intended to explain a preferred embodiment of how to operate the device.

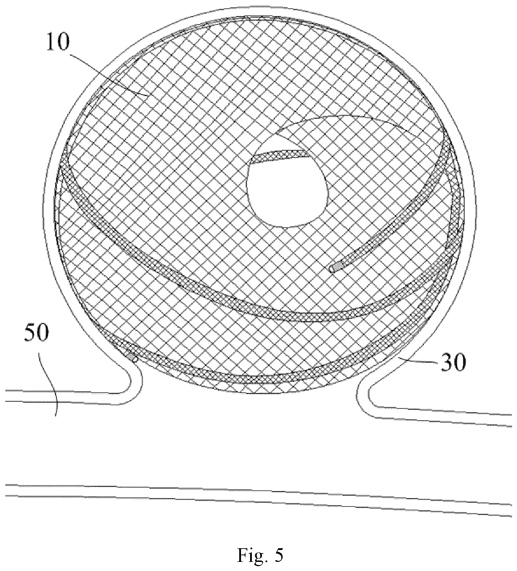

At first, the aneurysm occlusion device 10 is loaded into the catheter 40 for delivery. The aneurysm occlusion device 10 is loaded in the collapsed configuration, in which both the expandable structure 11 and the guide structure 12 are elongated preferably into linear shapes, resulting in an overall radial size of the device that is small enough to allow the device to be accommodated in the catheter 40 that has a small inner diameter. Optionally, the inner diameter of the catheter 40 may be 0.017 inches, 0.021 inches or 0.027 inches. After that, as shown in Fig. 4, a distal end of the catheter 40 is held stationary in the vicinity of the aneurysm 30, and the aneurysm occlusion device 10 is then released. In the release process, the push pod 20 may be manipulated to distally push the device 10 or proximally retract the catheter 40 relative to the device 10 to first release in the aneruysm 30 the guide structure 12, which then spirals in the aneurysm into the predetermined shape. Since the guide structure 12 is a slim flexible spiral structure, it encounters less friction in the aneurysm and thus can easily recover the spiral shape. With the aneurysm occlusion device 10 being further pushed, release of the expandable structure 11 in the aneruysm 30 starts. Under the guidance of the guide structure 12, the expandable structure 11 continues spiraling. In this process, the outer diameter of the expandable structure 12 increasingly expands until the completely expanded configuration is reached, as shown in Fig. 5. At this time, the outer surface of the largest spiral turn in the aneurysm occlusion device 10 covers the aneurysm neck from an inner side thereof, and the whole device stably spirals within the aneurysm 30, achieving stable, compliant packing. Finally, after desired packing quality is confirmed, the push pod 20 may be electrically detached from the expandable structure 11, and the catheter 40 and push pod 20 may be withdrawn, ending the occlusion process for the aneurysm 30.

With continued reference to Fig. 5, after the completion of the occlusion process, a distal end of the aneurysm occlusion device 10 (i.e., the distal end of the guide structure 12) is wrapped within the expandable structure 11 and thus has no impact on the aneurysm wall. Moreover, the proximal end 112 of the expandable structure 11 is confined between the outer surface of the largest spiral turn in the expandable structure 11 and the aneurysm wall so as to be oriented in parallel to the aneurysm wall and therefore has little impact on the aneurysm wall. In addition, the only portion contacting the aneurysm wall is a braided dense mesh. This enables the aneurysm wall to be more uniformly stressed and less damaged. In particular, the three-dimensional spiral shape of the expandable structure 11 may be composed of multiple spiral turns arranged at different radial locations. These spiral turns can provide multiple barriers in the aneurysm, which can better block blood flow and facilitate thrombosis, thus accelerating embolization of the aneurysm. With additional reference to Fig. 6, it is the outer surface of the largest spiral turn in the expandable structure 11 that closes the aneurysm neck opening 31 by fitting against the inner wall of the aneurysm 30. This imparts improved stability to the device. Further, it is unlikely for a proximal end of the device to herniate into the parent blood vessel 50 (see Fig. 5) and affect endothelialization over the aneurysm neck. Thus, healing of the aneurysm neck opening can be accelerated, and good embolization quality can be obtained.

Alternatively, only the expandable structure 11 but not the guide structure 12 may be provided while still achieving occlusion of the aneurysm 30. Preferably, the guide structure 12 is provided at the distal end of the expandable structure 11. This is advantageous in that, under the guidance of the guide structure 12, the expandable structure 11 can more easily recover the predetermined shape in the aneurysm and more easily achieve occlusion of the aneurysm neck.

Further, the guide structure 12 is preferred to be radiopaque. For example, it may be entirely made of a radiopaque material. The present invention is not limited to any particular radiopaque material of the guide structure 12, and for example, platinum (Pt), platinum-iridium (Pt-Ir) alloys, gold (Au), platinum-tungsten (Pt-W) alloys and the like can be suitably used. Alternatively, the guide structure 12 may be formed of a non-radiopaque material, such as a nickel-titanium alloy or stainless steel. In one embodiment, it may be obtained by making an elongate linear member through densely winding a metal wire on a metal core bar into a primary coil and then shaping the primary coil (also called a coil) into the guide structure 12 that assumes the spiral shape when in the expanded configuration. In another embodiment, the spiral-shaped guide structure 12 may be obtained by cutting a metal tube into a flexible mesh tube, stretching the flexible mesh tube into an elongate shape and then subjecting it to a shaping process. In yet another embodiment, the spiral-shaped guide structure 12 may be obtained by stretching a preformed braided tube into an elongate shape and then subjecting it to a shaping process. In further embodiments, the fabrication may involve directly obtaining an elongate linear member without a stretching process and then shaping the linear member into a spiral shape. It would be also appreciated that the expandable structure 11 and the guide structure 12 may be integrally braided.

Further, the distal end 122 of the guide structure 12 is preferred to be smooth. For example, a light-curable adhesive may be applied to the distal end 122 and then cured there to form a smooth dome-shaped tip, which can reduce damage caused to the aneurysm wall. Furthermore, a distal radiopaque ring 123 may be attached to the distal end 122 of the guide structure 12. On an aspect, the radiopacity of the distal radiopaque ring 123 under X-ray fluoroscopy allows location of the distal end 122 of the guide structure 12. On another aspect, the distal end 122 of the guide structure 12 can be confined and secured within the distal radiopaque ring 123 thus cause even less damage to the aneurysm wall. Alternatively, the distal end 122 of the guide structure 12 may be secured using an adhesive to cause less damage to the aneurysm wall. In this case, the distal radiopaque ring 123 may be additionally provided, entailing double protection.

The expandable structure 11 is preferably formed of a spirally wound braided tube. Preferably, the braided tube is formed of 48-144 filaments with a diameter of 0.0005-0.002 inches. In this way, a dense mesh with a high mesh opening density can be constructed, which can effectively block blood flow and promote thrombosis in the aneurysm and allows better coverage of the aneurysm neck and more uniform stressing of the aneurysm wall, thus additionally reducing the risk of rupture of the aneurysm. Materials suitable for fabrication of the filaments may include shape memory materials, which may be metal materials with shape memory properties, such as nickel-titanium (Ni-Ti) alloys, nickel-titanium-cobalt alloys (Ni-Ti-Co), double-layer composite metal wires (e.g., Ni-Ti@Pt), etc. Suitable materials for the filaments may also include polymer materials with certain shape recovery properties, such as polydioxanone (PDO), poly(l-lactide-co-ε-caprolactone) (PLC), polyurethane (PU), amorphous polynorbornene or combinations thereof. Making the filaments out of a shape memory metal material or a polymer material with certain shape recovery properties can imparts shape memory properties to the braided mesh, which can facilitate the recovery of the original shape. Preferably, the expandable structure 11 is braided from radiopaque filaments. Alternatively, the expandable structure 11 may be braided from both radiopaque and non-radiopaque filaments. With this design, the expandable structure 11 is itself radiopaque under X-ray fluoroscopy, while exhibiting sufficient resilience, which enables the expandable structure 11 to have strong ability to recover and maintain its original shape, thereby dispensing with the use of a separate radiopaque component and increasing compliance of the expandable structure 11. The present invention is not limited to any particular radiopaque material of the radiopaque filaments, and for example, platinum (Pt), platinum iridium alloys (Pt-Ir), gold (Au), platinum-tungsten (Pt-W) alloys and the like can be suitably used.

The guide structure 12 may have one or more spiral turns, preferably 1-6 spiral turns, more preferably 1-3 spiral turns. Considering that an excessive outer diameter of the spiral shape of the guide structure 12 tends to adversely affect the advancement or expansion of the expandable structure 11, an outer diameter of the largest spiral turn in the guide structure 12 in the expanded configuration is limited to not exceed an outer diameter of a first spiral turn in the expandable structure 11 proximal to the distal end thereof. For example, when the guide structure 12 has a plurality of spiral turns, an outer diameter of a first one of the spiral turns in the guide structure 12 proximal to the distal end thereof is preferred to be smaller than an outer diameter of any other spiral turn in the guide structure 12, which is preferred to not exceed the outer diameter of the first spiral turn in the expandable structure 11 proximal to the distal end thereof. Moreover, the outer diameters of the other spiral turns in the guide structure 12 may be equal or not, and are preferred to be equal. Additionally, the outer diameter of the first spiral turn in the guide structure 12 proximal to the distal end thereof is not smaller than 2/3 of the outer diameter of any other spiral turn in the guide structure 12, which is preferred to be in turn not smaller than 2/3 of the outer diameter of the first spiral turn in the expandable structure 11 proximal to the distal end thereof. This design is advantageous in that, during the release and shape recovery of the expandable structure 11, it can provide a space for the packing by the expandable structure 11 and prevent the spiral turns in the guide structure 12 from occupying additional space and hence from affecting the release and shape recovery of the expandable structure 11 in the aneurysm. It would be appreciated that the first spiral turn of the guide structure 12 refers to its most distal spiral turn. It would be appreciated that, the outer diameter of the largest spiral turn refers to a maximum outer diameter of its axial projection. The largest spiral turn refers to a spiral turn with the largest outer diameter.

The number of spiral turns in the expandable structure 11 may be determined according to the actual size of an aneurysm to be treated. Fewer spiral turns make the expandable structure 11 suitable for use in the treatment of a smaller aneurysm, and more spiral turns enable it to be used in the treatment of a bigger aneurysm. In this embodiment, the spiral shape of the expandable structure 11 in the expanded configuration may have one or more spiral turns, optionally 1-3 spiral turns. Considering that when the number of spiral turns is greater than 3, the aneurysm occlusion device 10 will encounter increased resistance to pushing and advancement and greater friction during shape recovery in the aneurysm, which may make it difficult for the expandable structure 11 to recover the spiral shape, the spiral shape of the expandable structure 11 in the expanded configuration preferably has no more than 3 spiral turns, more preferably, 1-3 spiral turns. In this embodiment, in case of the spiral shape of expandable structure 11 in the expanded configuration having a plurality of spiral turns, the outer diameter of the first spiral turn in the expandable structure 11 proximal to the distal end thereof is preferably smaller than outer diameter(s) of the other spiral turn(s) in the expandable structure 11, which is/are preferably equal. This design is advantageous in facilitating shape recovery of the expandable structure 11 by minimizing resistance, and in providing sufficient support, which can further stabilize the aneurysm occlusion device 10 and ensure a space for packing with an increased packing density. Preferably, the outer diameter of the first spiral turn in the expandable structure 11 proximal to the distal end thereof is not smaller than 2/3 of the outer diameter of any other spiral turn in the expandable structure 11. It would be appreciated that the first spiral turn of the expandable structure 11 refers to its most distal spiral turn.

Further, in one embodiment, as shown in Fig. 1, before being implanted into a patient's body, the guide structure 12 assumes a three-dimensional or planar spiral shape having 1.5 spiral turns when in the expanded configuration. Moreover, the expandable structure 11 is a three-dimensional spiral-shaped mesh structure having 1 spiral turn. In this case, the proximal end 112 and the distal end 111 of the expandable structure 11 are located at the same radial position (i.e., a line connecting the proximal end 112 and the distal end 111 is parallel to the axis of spiral of the expandable structure 11) on the same side of the axis of spiral. In addition, the expandable structure 11 and the guide structure 12 spiral in opposite directions, and the outer diameter of the first spiral turn in the guide structure 12 proximal to the distal end thereof is slightly smaller than, but is preferred to be not smaller than 2/3 of, an outer diameter of a second one of the spiral turns therein, which does not exceed, but is preferred to be not smaller than 2/3 of, the outer diameter of the first spiral turn in the expandable structure 11 proximal to the distal end thereof. Further, in the expanded configuration, a maximum outer diameter D1 of (an inner cavity defined by) the expandable structure 11 is preferably not smaller than 1/4, for example, 1/3-1/2, of the outer diameter D2 of the largest spiral turn therein. In this embodiment, the three-dimensional or planar spiral-shape guide structure 12 can guide the three-dimensional spiral-shaped expandable structure 11 to spiral in the aneurysm into its predetermined shape while reducing damage caused by the distal end of the expandable structure 11 to the aneurysm wall.

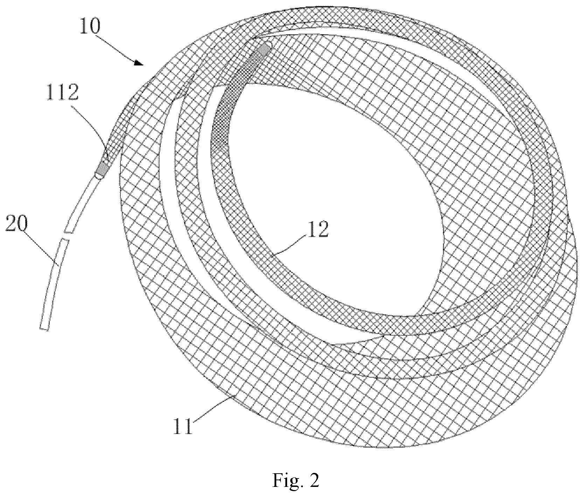

Alternatively, in another embodiment, as shown in Fig. 2, before being implanted into a patient's body, the guide structure 12 assumes a three-dimensional spiral shape having 2 spiral turns when in the expanded configuration. Moreover, the expandable structure 11 is a three-dimensional spiral-shaped mesh structure having 1 spiral turn. In this case, the proximal end 112 and the distal end 111 of the expandable structure 11 are located at the same radial position (i.e., a line connecting the proximal end 112 and the distal end 111 is parallel to the axis of spiral of the expandable structure 11, and projections of the ends on a horizontal plane coincide with each other) on the same side of the axis of spiral. In addition, the expandable structure 11 and the guide structure 12 spiral in the same direction, and the axes of spiral of them are preferably coincident with each other. In addition, the outer diameter of the first spiral turn in the guide structure 12 proximal to the distal end thereof is slightly smaller than, but is preferred to be not smaller than 2/3 of, an outer diameter of a second one of the spiral turns therein, and the outer diameter of the second spiral turn (i.e., the largest spiral turn) in the guide structure 12 does not exceed, but is preferred to be not smaller than 2/3 of, the outer diameter of the first spiral turn in the expandable structure 11 proximal to the distal end thereof. Further, in the expanded configuration, the maximum outer diameter D1 of the expandable structure 11 is preferably not smaller than 1/4, for example, 1/3-1/2, of the outer diameter D2 of the largest spiral turn therein. In this embodiment, the three-dimensional spiral-shape guide structure 12 can guide the three-dimensional spiral-shaped expandable structure 11 to spiral in the aneurysm into its predetermined shape.

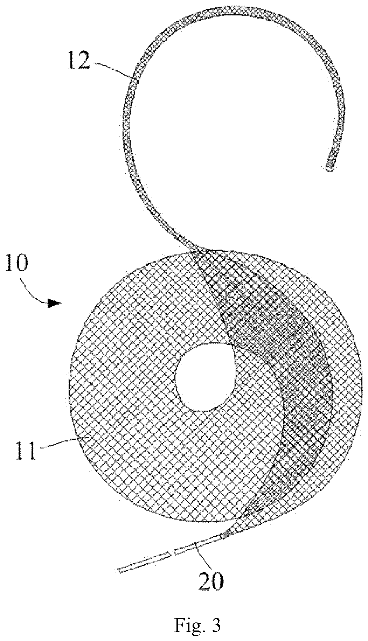

Still alternatively, in a further embodiment, as shown in Fig. 3, before being implanted into a patient's body, the guide structure 12 assumes a planar or three-dimensional spiral shape having 1 spiral turn when in the expanded configuration. Moreover, the expandable structure 11 is a three-dimensional spiral-shaped mesh structure having 1 spiral turn. In this case, the expandable structure 11 and the guide structure 12 spiral in opposite direction, and the axes of spiral of them does not coincide with each other. Further, in the expanded configuration, the outer diameter of the largest spiral turn in the guide structure 12 does not exceed, but is not smaller than 2/3 of, the outer diameter of the first spiral turn in the expandable structure 11 proximal to the distal end thereof. Furthermore, in the expanded configuration, the maximum outer diameter D1 of the expandable structure 11 is preferably not smaller than 1/4, for example, 1/3-1/2, of the outer diameter D2 of the largest spiral turn therein.

It would be appreciated that, in the expanded configuration, the expandable structure 11 may also have a plurality of spiral turns, for example, 1.5, 2, 2.5 or 3 spiral turns. The expandable structure 11 with more spiral turns can more completely pack the aneurysm and be used in the treatment of a bigger aneurysm.

As noted above, the maximum outer diameter D1 of the expandable structure 11 is preferably not smaller than 1/4, more preferably, 1/3-1/2, of the outer diameter D2 of the largest spiral turn therein. This is advantageous in ensuring sufficient friction between the outer surface of the largest spiral turn in the expandable structure 11 and the aneurysm wall, which prevents easy displacement of the aneurysm occlusion device 10 and disperses the support from the aneurysm occlusion device 10 to avoid the aneurysm wall from being damaged due to excessive local pressure. Additionally, it can be ensured that the outer surface of the largest spiral turn in the expandable structure 11 covers the aneurysm neck as much as possible to accelerate thrombosis in the aneurysm. In this embodiment, the outer diameter of the largest spiral turn in the expandable structure 11 is determined principally according to the size of the aneurysm to be treated. For example, the maximum outer diameter D1 of the expandable structure 11 may be 2.0-8.0 mm, and the outer diameter D2 of the largest spiral turn therein may be 3.0-25 mm.

Additionally, a proximal radiopaque ring 13 may be attached to the proximal end 112 of the expandable structure 11. On an aspect, the radiopacity of the proximal radiopaque ring 13 under X-ray fluoroscopy allows location of the proximal end 112 of the expandable structure 11 and increases overall radiopacity of the entire device. On another aspect, end portions of filaments in the expandable structure 11 can be hidden within the proximal radiopaque ring 13 and thus cause less damage to the aneurysm wall. Further, another radiopaque ring (not shown) may be attached to the distal end 111 of the expandable structure 11. On an aspect, the radiopacity of the radiopaque ring provided at the distal end 111 of the expandable structure 11 under X-ray fluoroscopy allows location of the distal end 111 of the expandable structure 11 and increases the overall radiopacity of the entire device. On another aspect, end portions of filaments in the expandable structure 11 can be hidden within the distal radiopaque ring and thus cause less damage to the aneurysm wall. Optionally, the proximal radiopaque ring 13 may be attached with an adhesive to the proximal end 112 of the expandable structure 12. Similarly, the other radiopaque ring may be attached with an adhesive to both the distal end 111 of the expandable structure 12 and the proximal end 121 of the guide structure 12. It would be appreciated that, when the expandable structure 11 itself is radiopaque, the proximal radiopaque ring 13 and the other radiopaque ring may be either provided or not. It would be also appreciated that it is possible to provide either or both of the distal radiopaque ring 123 and proximal radiopaque ring 13.

In this embodiment, a cross-sectional area of the expandable structure 11 is preferred to increase and then decrease from the proximal end 112 to the distal end 111. That is, the outer diameter of the three-dimensional mesh of the expandable structure 11 is not constant. Additionally, the expandable structure 11 includes, sequentially joined side by side axially from the distal end 111 to the proximal end 112, a distal section 113, a middle section 114 and a proximal section 115. The distal section 113 preferably has a cross-sectional area (or outer diameter) increasing from the distal end 111 to the middle section 114, and/or the proximal section 115 preferably has a cross-sectional area (or outer diameter) increasing from the proximal end 112 to the middle section 114. In other embodiments, the cross-sectional area of the expandable structure 11 may repeatedly increase and decrease from the proximal end 112 to the distal end 111. That is, it may repeatedly widen and narrow. This design can facilitate configuration of the expandable structure 11 into a fusiform shape (swollen at the middle and tapering to each end), which makes it easy to be compressed into a smaller size. Moreover, it enables the aneurysm occlusion device to be more flexible and pushed and advanced while experiencing less resistance and exerting a reduced impact on the aneurysm wall. Additionally, in this design, the distal section 113 can further function as a guide means, which facilitates shape recovery of the expandable structure 11. The expandable structure 11 may have a greater mesh opening density at both the distal section 113 and the proximal section 115. In particular, a greater mesh opening density of the distal section 113 enables the aneurysm occlusion device 10 to have increased strength, better support stability within the spiral shape and higher resistance to displacement. The present invention is not limited to any particular mesh opening density distribution of the middle section 114 of the expandable structure 11, and for example, it may have either a uniform or non-uniform mesh opening density.

In embodiments of the present invention, there is also provided an aneurysm occlusion system including the aneurysm occlusion device 10 and the catheter 40. The expandable structure 11 is compressed within the catheter 40 and, after being released from the catheter 40, recovers the expanded configuration in which it forms the spiral shape.

At last, it is to be noted that, although a few preferred embodiments of the present invention have been described above, the scope of the invention is in no way limited to these embodiments disclosed hereinabove. For example, the present invention is not limited to any particular number of spiral turns in the expandable structure in the expanded configuration, or any particular number of spiral turns in the guide structure in the expanded configuration, or any particular outer diameter of the largest spiral turn in the expandable structure in the expanded configuration.

Thus, embodiments of the present invention provide an aneurysm occlusion device capable of easier occlusion of the neck of an aneurysm while not exerting any impact on a wall of the aneurysm at its distal or proximal end, thereby avoiding the risk of rupture of the aneurysm, increasing coverage of the aneurysm neck and facilitating thrombosis in the aneurysm. Additionally, the problem that a proximal anchor of the expandable structure is located around a center of an opening of the aneurysm neck and may herniate into the parent blood vessel can be circumvented. In this way, endothelialization of the aneurysm neck can be accelerated, and the risk of vascular stenosis can be avoided.

The description presented above is merely that of a few preferred embodiments of the present invention and does not limit the scope thereof in any sense. Any and all changes and modifications made by those of ordinary skill in the art based on the above teachings fall within the scope as defined in the appended claims.

## Claims

1. An aneurysm occlusion device, comprising an expandable structure and a guide structure, the expandable structure being a three-dimensional mesh, the expandable structure having an expanded configuration, in which the expandable structure forms a spiral shape by spiraling from a distal end to a proximal end thereof, and a collapsed configuration for delivery into an aneurysm from a blood vessel,
the guide structure provided at the distal end of the expandable structure, the guide structure having a proximal end connected to the distal end of the expandable structure, the guide structure having an expanded configuration, in which the guide structure forms a spiral shape by spiraling from a distal end to the proximal end thereof, and a collapsed configuration for delivery into the aneurysm from the blood vessel.

2. The aneurysm occlusion device according to claim 1, wherein the guide structure is an elongated member spiraling from a distal end to a proximal end thereof into a three-dimensional spiral shape.

3. The aneurysm occlusion device according to claim 1, wherein the guide structure spirals in a same direction as the expandable structure.

4. The aneurysm occlusion device according to claim 1, wherein an axis of spiral of the guide structure coincides with an axis of spiral of the expandable structure.

5. The aneurysm occlusion device according to any one of claims 1 to 4, wherein a distal radiopaque ring is fixedly connected to the distal end of the guide structure, and/or a proximal radiopaque ring is fixedly connected to the proximal end of the expandable structure.

6. The aneurysm occlusion device according to any one of claims 1 to 4, wherein a cross-sectional area of the expandable structure increases and then decreases from the proximal end to the distal end thereof.

7. The aneurysm occlusion device according to claim 6, wherein the expandable structure comprises a proximal section, a middle section and a distal section which are connected axially in sequence,
wherein the proximal section has a cross-sectional area gradually increasing from a proximal end to a distal end thereof, and/or the distal section has a cross-sectional area gradually increasing from a distal end to a proximal end thereof.

8. The aneurysm occlusion device according to any one of claims 1 to 4, wherein a cross-sectional area of the expandable structure repeatedly increases and then decreases from the proximal end to the distal end thereof.

9. The aneurysm occlusion device according to any one of claims 1 to 4, wherein a maximum outer diameter of the expandable structure in the expanded configuration thereof is not smaller than 1/4 of an outer diameter of a largest spiral turn in the expandable structure.

10. The aneurysm occlusion device according to claim 9, wherein the maximum outer diameter of the expandable structure in the expanded configuration thereof is 1/3-1/2 of the outer diameter of the largest spiral turn in the expandable structure.

11. The aneurysm occlusion device according to any one of claims 1 to 4, wherein the expandable structure is formed by braiding 48-144 filaments which are made of a shape memory material and have a diameter of 0.0005-0.002 inches.

12. The aneurysm occlusion device according to claim 11, wherein the expandable structure is formed by braiding radiopaque filaments, or the expandable structure is formed by braiding both radiopaque and non-radiopaque filaments.

13. The aneurysm occlusion device according to any one of claims 1 to 4, wherein the spiral shape of the expandable structure in the expanded configuration thereof has one spiral turn or a plurality of spiral turns,
wherein in case of the plurality of spiral turns, a first one of the spiral turns in the expandable structure proximal to the distal end thereof has an outer diameter smaller than an outer diameter of each spiral turn therein other than the first spiral turn.

14. The aneurysm occlusion device according to claim 13, wherein in case of the plurality of spiral turns, the outer diameter of the first spiral turn is not smaller than 2/3 of the outer diameter of each spiral turn other than the first spiral turn.

15. The aneurysm occlusion device according to claim 14, wherein in case of the plurality of spiral turns, the outer diameter(s) of other spiral turn(s) than the first spiral turn in the expandable structure is/are equal.

16. The aneurysm occlusion device according to any one of claims 1 to 4, wherein the spiral shape of the guide structure in the expanded configuration thereof has one spiral turn or a plurality of spiral turns,
wherein in case of the plurality of spiral turns, a first one of the spiral turns in the guide structure proximal to the distal end thereof has an outer diameter smaller than an outer diameter of each spiral turn therein other than the first spiral turn.

17. The aneurysm occlusion device according to claim 16, wherein in case of the plurality of spiral turns, the outer diameter of each spiral turn other than the first spiral turn in the guide structure does not exceed the outer diameter of the first spiral turn in the expandable structure proximal to the distal end thereof.

18. The aneurysm occlusion device according to claim 16, wherein in case of the guide structure having the plurality of spiral turns, the outer diameter of the first spiral turn in the guide structure proximal to the distal end thereof is not smaller than 2/3 of the outer diameter of each spiral turn therein other than the first spiral turn.

19. The aneurysm occlusion device according to claim 18, wherein in case of the guide structure having the plurality of spiral turns, the outer diameter(s) of the other spiral turn(s) than the first spiral turn in the guide structure is/are equal.

20. The aneurysm occlusion device according to claim 19, wherein in case of the guide structure having the plurality of spiral turns, the outer diameter of each spiral turn other than the first spiral turn in the guide structure is not smaller than 2/3 of the outer diameter of the first spiral turn in the expandable structure proximal to the distal end thereof.

21. The aneurysm occlusion device according to any one of claims 1 to 4, wherein the expandable structure and the guide structure are integrally braided.

22. A therapeutic apparatus for aneurysm occlusion, comprising the aneurysm occlusion device according to any one of claims 1 to 21 and a push pod connected to the proximal end of the expandable structure.

23. The therapeutic apparatus for aneurysm occlusion according to claim 22, wherein the push pod extends in a direction tangential to a spiral contour of the spiral shape of the expandable structure in the expanded configuration thereof.

24. An aneurysm occlusion system, comprising the aneurysm occlusion device according to any one of claims 1 to 21 and a catheter, wherein the expandable structure is compressed in the catheter and, after being released from the catheter, recovers the expanded configuration in which the expandable structure forms the spiral shape.

25. The aneurysm occlusion system according to claim 24, wherein the catheter has an inner diameter of 0.017 inches, 0.021 inches or 0.027 inches.
